# EUROPÄISCHE PATENTSCHRIFT

(11) **EP 3 175 018 B1**
(45) Veröffentlichungstag und Bekanntmachung des Hinweises auf die Patenterteilung: **26.09.2018**
(21) Anmeldenummer: 15759628.9
(22) Anmeldetag: 31.07.2015
(51) Int. Cl.: C23F 1/26, A61L 27/06, A61F 2/30, B08B 7/00, C25F 3/08, H01L 21/311, H01L 21/3213

(54) **METALLISCHES WERKSTÜCK AUS TITAN UND/ODER TITAN-LEGIERUNG UND/ODER NICKEL-TITAN-LEGIERUNGEN SOWIE NITINOL MIT PORIGER OBERFLÄCHE UND HERSTELLUNGSVERFAHREN**
METALLIC WORKPIECE OF TITANIUM AND/OR A TITANIUM ALLOY AND/OR NICKEL-TITANIUM ALLOYS AND ALSO NITINOL WITH A POROUS SURFACE AND PRODUCTION PROCESS
PIÈCE MÉTALLIQUE, À SURFACE POREUSE, CONSTITUÉE DE TITANE ET/OU D'ALLIAGE DE TITANE ET/OU D'ALLIAGES DE NICKEL-TITANE AINSI QUE DE NITINOL ET PROCÉDÉ DE FABRICATION

(30) Priorität: 31.07.2014 DE 102014110922
(43) Veröffentlichungstag der Anmeldung: 07.06.2017
(73) Patentinhaber: Christian-Albrechts-Universität zu Kiel, 24118 Kiel (DE)
(72) Erfinder: BAYTEKIN-GERNGROSS, Melike, 24105 Kiel (DE); GERNGROSS, Mark-Daniel, 24105 Kiel (DE); ADELUNG, Rainer, 24147 Kiel (DE); CARSTENSEN, Jürgen, 24146 Kiel (DE)
(74) Vertreter: Hansen, Jochen
(86) Internationale Anmeldenummer: PCT/DE2015/100322
(87) Internationale Veröffentlichungsnummer: WO 2016/015720

(56) Entgegenhaltungen:
- WO-A2-2010/068468
- US-A1- 2009 114 619
- AITA H ET AL: "The effect of ultraviolet functionalization of titanium on integration with bone", BIOMATERIALS, ELSEVIER SCIENCE PUBLISHERS BV., BARKING, GB, Bd. 30, Nr. 6, 1. Februar 2009 (2009-02-01), Seiten 1015-1025, XP025840046, ISSN: 0142-9612, DOI: 10.1016/J.BIOMATERIALS.2008.11.004 [gefunden am 2008-11-29]
- IWASA F ET AL: "Enhancement of osteoblast adhesion to UV-photofunctionalized titanium via an electrostatic mechanism", BIOMATERIALS, ELSEVIER SCIENCE PUBLISHERS BV., BARKING, GB, Bd. 31, Nr. 10, 1. April 2010 (2010-04-01) , Seiten 2717-2727, XP026882478, ISSN: 0142-9612, DOI: 10.1016/J.BIOMATERIALS.2009.12.024 [gefunden am 2010-01-30]

## Beschreibung

Die vorliegende Erfindung betrifft das Gebiet der Erzeugung von Oberflächen auf metallischen Werkstücken mit verbesserter Haftfähigkeit für organische Polymere und/oder biologische Materialien und/oder keramische Materialien.

Die Erfindung betrifft ein oberflächenbehandeltes metallisches Werkstück aus Titan und/oder Titan-Legierungen mit Titan als Hauptbestandteil und/oder Nickel-Titan-Legierungen sowie Nitinol, wobei das Metall an der behandelten Oberfläche frei von Einschlüssen, Ausscheidungen anderer Metalle, Anlagerungen von Alkali-, Erdalkalimetallen und/oder Aluminium, intermetallischen Phasen, und/oder mechanisch stark defektreichen Bereichen ist, und die Oberfläche über eine erste Rauigkeit und eine zweite Rauigkeit verfügt, wobei die erste Rauigkeit durch Vertiefungen in Form von Poren gegeben ist, wobei die Poren- einen Durchmesser im Bereich zwischen 0,5 und 50 µm haben- in Richtung der Oberfläche offen und- in Richtung des Werkstücks geschlossen sind, und wenigstens ein Teil der Poren einen Hinterschnitt aufweisen und die zweite Rauigkeit durch statistisch verteilte Erhöhungen und Vertiefungen im Bereich von 100nm und weniger gegeben ist.

Ferner betrifft die Erfindung ein Herstellungsverfahren für ein oberflächenbehandelten Werkstück.

Es ist grundsätzlich bekannt, dass eine Erhöhung der Rauheit einer metallischen Werkstückoberfläche hilfreich sein kann, die Haftung von Deckschichten zu verbessern. Insbesondere können Gräben, Poren oder lokale Erhöhungen auf der Werkstückoberfläche Strukturen sein, die sich von einem fließfähigen Polymer, Präpolymer oder Monomer durchsetzen, auffüllen oder umschließen lassen, so dass der Polymerfilm nach seiner Aushärtung infolge einer mechanischen Verankerung fixiert ist.

In der Medizin ist die Verwendung von Implantaten mit porösen Oberflächen zur besseren Fixierung des umliegenden organischen Gewebes seit mehr als 20 Jahren bekannt.

Insbesondere wegen der hohen Biokompatibilität, der guten Korrosionsbeständigkeit und der geringen Toxizität sind Titan und seine Legierungen ein in der Medizin weit verbreitetes Material für Implantate.

Die zur erfolgreichen Osseointegration notwendige Rauheit des aus Titan oder seinen Legierungen bestehenden Implantates lässt sich durch Diffusionsschweißen, Sintern, Sandstrahlen, Plasmaspritzen, sukzessives Ätzen oder andere Verfahren erreichen.

Aus Hacking et al. "Acid-etched microtexture for enhancement of bone groth into porous-coated implants" [Journal of Bone and Joint Surgery 85, 1182] ist bekannt, das zur Ausbildung einer Mikrotextur Ätzen gegenüber dem Sintern vorteilhaft sein kann.

Aus der US 2007/0187253 A1 ist bekannt, beta (β) -Phasen aus Ti-6Al-4V-Legierungen oberflächennah herauszulösen, um so eine nanotopographische Oberfläche zu erzeugen. Das Verfahren ist auf die Strukturierung von Ti-6Al-4V-Legierungen beschränkt. Das beschriebene Verfahren ist ebenfalls auf das Herauslösen der β- Phase beschränkt, wird diese nicht ausgebildet, ist es nicht einsetzbar, des Weiteren verbleiben andere, nicht durch die β Phase gegebenen Verunreinigungen und mechanische Defekte, auf der Oberfläche und tragen so auch nicht zur günstigen Topographie bei.

Ein weiterer Nachteil des Verfahrens ist, dass für die Strukturierung der Ti-6Al-4V-Legierungsoberfläche der Einbau in eine elektrochemische Zelle mit entsprechender elektrischer Kontaktierung zwingend notwendig ist, dies bedeutet, dass die Kontaktierungsstelle des Werkstückes nicht in Kontakt mit dem Elektrolyten kommen darf, um definierte und reproduzierbare elektrochemische Bedingungen während der Prozesses zu gewährleisten und somit nicht das gesamte Werkstück oberflächenmodifiziert werden kann.

Aus der US2009/114619 A1 ist bekannt, ein photochemisches Ätzensverfahren für metallische Bauteile aus Titan oder Titan-Legierung, wobei das chemische Ätzen in Gegenwart einer sauren Lösung bei gleichzeitigen UV-Beleuchten durchgeführt wird. WO 2010/0684468 A2 beschreibt ein Ätzensverfahren von metallischen Oberflächen aus Titan oder Titan-Legierungen, welches ein chemisches Ätzen in Gegenwart einer sauren Lösung, die Schwefelsäure enthält, und danach UV-Beleuchten bis zum 48 Stunden, umfasst. Es ist es Aufgabe der Erfindung metallische Werkstücke zur Verfügung zu stellen, die über eine besonders günstige Topographie verfügen.

Ferner ist es Aufgabe der Erfindung metallische Werkstücke mit einer günstigen Topographie aus den Metallen Titan, Zirkonium, Hafnium, Vanadium, Niob, Tantal und der Legierungen dieser Metalle sowie der unter dem Namen "Nitinol" bekannten Nickel-Titan-Legierung zur Verfügung zu stellen.

Es ist ebenfalls Aufgabe der Erfindung metallische Werkstücke zur Verfügung zu stellen, die an ihrer Oberfläche frei von Einschlüssen und Ausscheidungen anderer Metalle und/oder intermetallischer Phasen sowie defektierten Bereichen sind.

Darüber hinaus ist es Aufgabe der Erfindung ein Verfahren zur Strukturierung der Oberfläche zur Verfügung zu stellen, welches auch auf Werkstücke aus Titan und/oder Titanlegierungen verschiedener Zusammensetzungen sowie auch auf weitere Metalle und Legierungen anwendbar ist.

Des Weiteren ist es Aufgabe der Erfindung, ein Verfahren zur Verfügung zu stellen, dass neben der β-Phase auch weitere Verunreinigungen oder mechanische Defekte aus der Oberfläche entfernt und somit die Reinheit der Oberfläche erhöht.

Des Weiteren ist es Aufgabe der Erfindung ein Verfahren zur Verfügung zu stellen, dass es ermöglicht, komplette Werkstücke, auch mit komplexer Form, zu strukturieren.

Die Aufgabe der Erfindung wird gelöst durch die Bereitstellung eines metallischen Werkstücks aus den Metallen Titan, Zirkonium, Hafnium, Vanadium, Niob, Tantal und/oder deren Legierungen, sowie der unter dem Namen "Nitinol" bekannten Nickel-Titan-Legierung wobei das Metall an der behandelten Oberfläche weitgehend frei von Einschlüssen und oder Ausscheidungen anderer Metalle und oder intermetallischer Phasen sowie mechanisch stark defektreichen Bereichen (z.B. Versetzungsnestern) ist und wobei das Werkstück über eine Topographie verfügt, die gegeben ist durch eine erste und eine zweite Rauheit und wobei die erste Rauheit durch Vertiefungen in Form von Poren gegeben ist und wobei die Poren in Richtung der Oberfläche offen und in Richtung des Werkstücks geschlossen sind und wobei mindestens 25 %, bevorzugt 50% der Poren einen Hinterschnitt aufweisen und wobei die zweite Rauheit durch statistisch verteilte Erhöhungen und Vertiefungen im Bereich von 100nm und weniger gegeben ist.

Desweiteren wird die Aufgabe der Erfindung durch ein Verfahren zur Herstellung des erfindungsgemäßen Werkstücks gelöst welches die folgenden Schritte umfasst
i. Photochemisches Ätzen der Oberfläche in einem Elektrolyten unter Beleuchtung
ii. Chemisches Ätzen in saurer Lösung

Als Werkstück eignen sich die Metalle Titan, Zirkonium, Hafnium, Vanadium, Niob, Tantal, und/oder deren Legierungen. Beispiele für geeignete Titanlegierungen finden sich in Materials Properties Handbook: Titanium Alloys, R. Boyer, G. Welsch, and E. W. Collings, eds. ASM International, Materials Park, OH, 1994. Besonders geeignet sind hier Titan grade 2, Ti-6Al-4V (grade 5) und Derivate von dieser Legierung (z.B. grades 23-25, grade 29), Ti-3Al-2.5V (grade 9) und Derivate von diesen Legierungen wie (z.B. grade 18 und grade 28) sowie NiTi (Nitinol). Im Fall von Niob wären geeignete Legierungen z.B. C-103, C-129Y, C3009, Cb 752, FS85 und Nb1Zr.

Dabei ist es im Allgemeinen vorteilhaft, vor dem ersten Ätzschritt i das Werkstück von groben Verschmutzungen und Fett/Öl zu reinigen. Die Reinigung erfolgt auf einer dem Fachmann allgemein bekannter Art und Weise, beispielsweise durch Spülen mit wassermischbaren organischen Lösungsmitteln z.B. durch ein Acetonbad oder ein Bad bestehend aus 70% Isopropanol und 30% deionisiertem Wasser.

Während des photochemischen Ätzschrittes i wird dabei eine chemisch größtmögliche Oberflächen-Glattheit erzeugt. Als Elektrolyt kommt dabei eine stark oxidierende Säure in Frage.

Unter oxidierenden Säuren versteht der Fachmann allgemein solche Säuren, die neben dem aus den Protonen resultierendem Wirkungspotential noch an einer Redoxreaktion teilzunehmen. Das Anion der oxidierenden Säure oxidiert das Metall und wird dabei reduziert. Das Eintreten der oxidierenden Wirkung hängt dabei in der dem Fachmann bekannten Weise auch von der Konzentration der Säure und der Temperatur ab. Typischerweise kommen stark oxidierende Säuren aus der Gruppe der Sauerstoffsäuren. Bevorzugt ist die stark oxidierende Säure ausgewählt aus der Gruppe Schwefelsäure (H₂SO₄), Salpetersäure (HNO₃), Peroxomonoschwefelsäure (H₂SO₅), Chlorsäure (HClO₃), Perchlorsäure (HClO₄), Chromsäure (H₂CrO₄), Arsensäure (H₃AsO₄), Wasserstoffperoxid (H₂O₂) und/oder einer Kombination dieser Säuren und/oder einer oder einer Kombination dieser Säuren in Verdünnung mit Wasser (H₂O).

Die Beleuchtung kann mit LED Arrays z.B. basierend auf Enfis Uno Tag Arrays erfolgen. Die nominelle Bestrahlungsstärke (Radiant Flux) sollte dabei bevorzugt zwischen 200 und 450 mW. Die Wellenlängen können dabei im Bereich zwischen 190 und 780 nm liegen.

Die Form des Probenbehälters und der Abstand zur Beleuchtungsquelle sind dabei in Abhängigkeit von der Probengeometrie so zu wählen, dass eine homogene Beleuchtung ermöglicht wird. Typischerweise wurde ein Abstand von 8 cm zwischen Probenoberfläche und LED-Array gewählt.

Der photochemische Ätzschritt i findet bei einer Temperatur zwischen 10°C und 50°C, bevorzugt zwischen 15°C und 40°C, besonders bevorzugt zwischen 20°C und 25°C (Raumtemperatur) statt.

Die Dauer des photochemischen Ätzschrittes i beträgt dabei 5 bis 30 Stunden, bevorzugt 10 bis 25 Stunden, besonders bevorzugt 15 bis 22 Stunden.

Nach dem photochemischen Ätzen wird die Probe mehrfach in dem Fachmann allgemein bekannter Art und Weise gespült, z.B. mit deionisiertem Wasser und anschließend getrocknet z.B. durch einfaches Lufttrocknen bevor sie in das Ätzbad ii gebracht wird.

Der chemische Ätzschritt ii dient der Herauslösung von Einschlüssen und/oder Ausscheidungen und/oder mechanisch defektreichen Bereichen und findet in saurer Lösung statt. Die saure Lösung ist dabei eine Kombination einer stark oxidierenden Säure und einer oxidauflösenden Säure.

Unter oxidauflösenden Säuren sind solche Säuren zu verstehen, die in der Lage sind, Metalloxide unter Bildung von Wasser in Metallsalze zu überführen. Bevorzugt sind die oxidauflösenden Säuren ausgewählt aus der Gruppe Salzsäure (HCl), Flusssäure (HF), Bromsäure (HBr), lodsäure (HI), einer Kombination dieser Säuren und/oder einer oder einer Kombination dieser Säuren in Verdünnung mit Wasser (H₂O).

Die Ätzlösung für Ätzschritt ii (Ätzlösung ii) kann dabei ausgewählt sein aus der Gruppe Schwefelsäure (H₂SO₄), Salzsäure (HCl), Flusssäure (HF) gegebenenfalls in Verdünnung mit Wasser

Die Ätzlösung basiert typischerweise auf 1:1 Mischung aus konzentrierter Schwefelsäure (96-98%) und konzentrierter Salzsäure (37 %) oder Flusssäure (40 %). Wahlweise kann hierbei auch das Verhältnis zwischen konzentrierter Schwefelsäure und konzentrierter Salzsäure / konz. Flusssäure variiert werden z.B. auf ein Verhältnis 1:2. Ebenso kann diese Ätzlösung ii durch Zugabe von deionisiertem Wasser verdünnt werden.

Der chemische Ätzschritt ii wird bevorzugt bei Raumtemperatur durchgeführt. Die Probe wird vorzugsweise direkt nach Herstellung der Ätzlösung in diese hineingegeben. Das Ätzbad wird typischerweise nicht gerührt, da es während der Ätzung zu Gasentwicklung kommt, wobei die Gasentwicklung zu einer Durchmischung der Ätzlösung führt. Das verwendete Volumen der Ätzlösung wird dabei so gewählt, dass die Ätzlösung im Überfluss vorhanden ist im Vergleich zum Probenvolumen und so das Probenvolumen vollständig benetzt wird. Der Ätzschritt ii findet in der Regel bei Raumtemperatur statt, ohne das Hinzuführen von externer Wärme, aber unter Verwendung der entstehenden Reaktionswärme.

Die Dauer des chemischen Ätzschrittes ii liegt zwischen 1 und 6 Stunden und kann sich durch Verdünnung des Ätzbades verlängern. Bei unverdünnter Ätzlösung ii beträgt die Dauer des Ätzschrittes ii bevorzugt 2 bis 4 Stunden, besonders bevorzugt 3 Stunden.

Nach dem chemischen Ätzschritt ii wird das oberflächenbehandelte Werkstück mehrfach in dem Fachmann allgemein bekannter Art und Weise gespült, z.B. mit deionisiertem Wasser und anschließend getrocknet z.B. durch einfaches Lufttrocknen.

Die erfindungsgemäßen Werkstücke weisen nach der zweistufigen Ätzbehandlung eine hohe Reinheit und Freiheit von mechanischen Defekten an der Oberfläche auf.

Nachfolgend werden Ausführungsbeispiele der Erfindung anhand der beiliegenden Abbildungen beschrieben.

Darin zeigen:

| | |
|---|---|
| Abb. 1 | eine Rasterelektronenmikroskopie REM erzeugte Aufnahme eines erfindungsgemäßen Werkstücks; |
| | |
| Abb. 2 | Rasterelektronenmikroskopie Top-Ansicht einer erfindungsgemäß behandelten Ti-3Al-2.5V Oberfläche, |
| | a) makroskopische Übersicht, |
| | b) mikroskopische Übersicht, |
| | c) gleicher Ausschnitt und gleicher Vergrößerung, aber Sekundärelektronen-Detektor und |
| | d) hohe Vergrößerung auf die Oberfläche mit schalenförmigen Vertiefungen mit Hinterschnitt; |
| | |
| Abb. 3 | EDX-Analyse bei 15 keV: Spektrum der Elementen-Verteilung auf der Ti-3Al-2.5V Oberfläche nach erfindungsgemäßer Prozessierung; |
| | |
| Abb. 4 | Rasterelektronenmikroskopie Draufsicht auf |
| | a) nach Strahlen und |
| | b) nach erfindungsgemäßer Prozessierung bei unterschiedlicher Vergrößerung; |
| | |
| Abb. 5 | EDX Elementen-Verteilung von Al, Ti und V sowie die dazugehörige Oberfläche, |
| | a) nach Strahlen und |
| | b) nach zusätzlicher erfindungsgemäßer Prozessierung; |
| Abb. 6 | EDX-Analyse bei 15 keV: Summenspektrum der Elementen-Verteilung auf der Ti-6Al-4V Oberfläche, |
| | a) nach Strahlen und |
| | b) nach zusätzlicher erfindungsgemäßer Prozessierung; |
| | |
| Abb. 7 | REM Draufsicht auf die Oberfläche einer Ti-6Al-4V Probe |
| | a) nach zusätzlicher erfindungsgemäßer Prozessierung (Ätzlösung ii mit Wasser verdünnt) |
| | b) in hoher Vergrößerung |
| | |
| Abb. 8 | REM Draufsicht auf die Oberfläche einer Ti-6Al-4V Probe |
| | a) nach zusätzlicher erfindungsgemäßer Prozessierung (Ätzlösung i mit Wasser verdünnt) |
| | b) in hoher Vergrößerung |
| | |
| Abb. 9 | REM Draufsicht auf die Oberfläche von einem NiTi Legierungsdraht, |
| | a) vor der Prozessierung, |
| | b) in hoher Vergrößerung, |
| | c) nach dem ersten Ätzschritt i, |
| | d) in hoher Vergrößerung, |
| | e) nach kompletter erfindungsgemäßer Prozessierung und |
| | f) in hoher Vergrößerung; |
| | |
| Abb. 10 | EDX Elementen-Verteilung von Ti und Ni sowie die dazugehörige Oberfläche, |
| | a) unbehandelt und |
| | b) nach vollständiger erfindungsgemäßer Prozessierung; |
| | |
| Abb. 11 | EDX-Analyse bei 15 keV: Summenspektrum der Elementen-Verteilung auf der Ti-6Al-4V Oberfläche, |
| | a) unbehandelt im Querschnitt, |
| | b) unbehandelt an der Oberfläche, |
| | c) nach dem ersten Ätzschritt i und |
| | d) nach vollständiger erfindungsgemäßer Prozessierung; |
| Abb. 12 | REM Draufsicht auf die Ti grade 2 Oberfläche nach |
| | a) Ätzschritt i für 20 h und |
| | b) kompletter erfindungsgemäßer Prozessierung, |
| | c) in hoher Vergrößerung und |
| | d) im Querschnitt. Bei unterschiedlicher Vergrößerung; |
| | |
| Abb. 13 | EDX Analyse bei 15 keV: Element-Verteilung von Ti, AI und V sowie die dazugehörige Proben Oberfläche (Ti grade 2, 99,6% Reinheit) nach vollständiger erfindungsgemäßer Prozessierung. |
| | |
| Abb. 14 | REM Draufsicht auf die Ti grade 2 Oberfläche nach |
| | a) Ätzschritt i für 20 h (Beleuchtung im UV-Bereich) und |
| | b) kompletter erfindungsgemäßer Prozessierung |

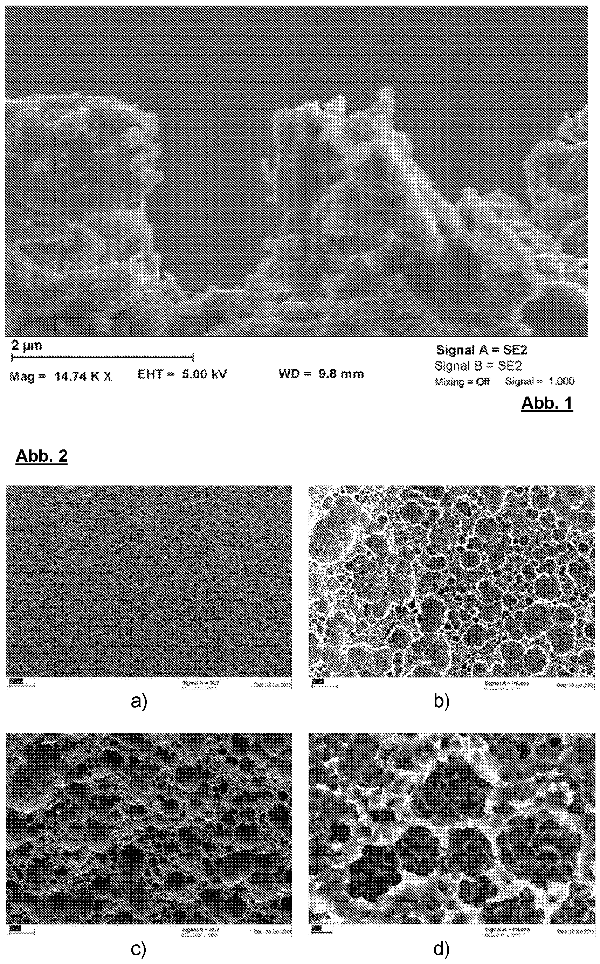

Abbildung 1 zeigt die mittels Rasterelektronenmikroskopie REM erzeugte Aufnahme eines erfindungsgemäßen Werkstücks. Es ist deutlich die erste Rauheit in Form von Poren, die in Richtung der Oberfläche offen und in Richtung des Werkstücks geschlossen sind zu erkennen. Es ist eine Pore mit deutlichem Hinterschnitt zu sehen, die im Inneren einen Durchmesser hat, der größer ist als der Durchmesser der Porenöffnung. Des Weiteren ist die zweite Rauheit durch statistisch verteilte Erhöhungen und Vertiefungen im Bereich von 100nm und weniger zu erkennen.

Aufgrund dieser Topographie bestehend aus einer Rauheit im Nanobereich und der Poren mit einem Durchmesser von 0,5 µm bis 50 µm, bevorzugt 1 µm bis 40 µm, besonders bevorzugt 2 µm bis 20 µm mit Hinterschnitt sind die erfindungsgemäßen Werkstoffe hervorragend für den haftenden Verbund mit anderen Materialien geeignet. So können die Werkstücke im Bereich der medizinischen Implantat-Technologie eingesetzt werden.

Die erfindungsgemäßen Werkstücke können Anwendung finden z.B. für Zahnimplantate oder künstliche Hüftgelenke. Für Zahnimplante ist die Osseointegration des Ti-Implantates von großer Wichtigkeit für die Langzeitstabilität des Implantates. Die Topographie aus Poren mit Hinterschnitt sowie die Nanorauhigkeit in den Poren sorgen für eine hervorragende mechanische Verzahnung zwischen Knochen und Implantat.

Mit der Ti-Oberflächenstrukturierung kann auch eine Aufreinigung der Ti-Oberfläche erfolgen, was z.B. zu einer geringeren Abgabe von Legierungs-Metall-Ionen aus dem Implantat, z.B. AI oder V-Ionen, führt. Dies ist insbesondere z.B. für künstliche Hüft und Kniegelenke von Bedeutung.

In der Technik sind die erfindungsgemäßen Werkstücke insbesondere im Bereich der Komposit-Materialien von Bedeutung, da es hier stark auf den Verbund der verschiedenen Materialien ankommt. So können die erfindungsgemäßen Werkstücke für Komposit-Turbinenschaufeln verwendet werden. Das Komposit besteht hierbei aus einer Schichtfolge von faserverstärktem Polymer und Ti-Blechen, die miteinander verbunden sind. Für diese Verbindung ist eine optimale Haftung zwischen Polymer und Ti-Blech von größter Wichtigkeit. Die Poren-Hinterschnitte und die Nanorauhigkeit der Poren sorgen hier für die mechanische Verzahnung zwischen dem Ti-Blech und dem Polymer.

Die Allgemeinheit der Lehre nicht einschränkend soll die Erfindung hier an einigen Beispielen erläutert werden:

### Beispiel 1: Ti-3Al-2.5V

### Herstellungsprozess:

Die Probe (Turbinenschaufel) wurde sandgestrahlt und anschließend für 5 min in Aceton gereinigt und an Luft getrocknet. Anschließend wird die Probe für 24 h in konzentrierter H₂SO₄ bei Raumtemperatur ohne externe Temperierung unter Beleuchtung mit einem ENFIS Uno Tag Red LED-Array betrieben bei einer nominalen Bestrahlungsstärke von 400 mW (300 mA und einer Wellenlänge von 620 nm) im Abstand von etwa 8 cm von der Probenoberfläche geätzt. Nach diesem Schritt wurde die Probe mehrfach in deionisiertem Wasser gereinigt und an Luft getrocknet. Anschließend wird die Probe für 3h in kurz vorher frisch hergestellter Ätzlösung bestehend aus HCl und H₂SO₄ im Volumen-Verhältnis 1:1 ohne externe Temperierung geätzt. Danach wurde die Probe mehrfach in deionisiertem Wasser gereinigt und an Luft getrocknet. Die Volumina der jeweiligen Ätzlösung wurden derart gewählt, dass eine vollständige Benetzung der Probe gegeben ist. Die Ätzlösungen wurden während der Ätzung nicht gerührt.

Die Rasterelektronenmikroskopie-Aufnahmen (REM) in Abb. 2. zeigen die mikrostrukturierte Oberfläche der erfindungsgemäß behandelten Probe Ti-3Al-2.5V (Turbinenschaufeln). Abb. 2a zeigt, dass die Oberfläche homogen ohne extreme Rauigkeitsunterschiede ist. Die Vergrößerung der Oberfläche in Abb. 2b zeigt eine schalenförmige Strukturierung mit verschieden großen Schalendurchmessern, die von 2 µm bis etwa 20 µm reichen. Besonders im linken Teil von Abb. 2b sieht man das Ineinanderwachsen dieser Schalen, was zu noch größeren Schalen führt. Dieses Ineinanderwachsen lässt sich am besten in Abb. 2c sehen. Abb. 2d zeigt die Oberfläche in hoher Vergrößerung. Man sieht, dass die schalenartigen Vertiefungen kleine scharfkantige Submikro-Strukturen aufweisen. Die helleren/weißen Kanten um die schalenartigen Vertiefungen werden durch die Hinterschneidungen verursacht, die während des Ätzprozesses entstehen. Die Hinterschnitt-Struktur zusammen mit den kleinen scharfkantigen Submikro-Strukturen in und zwischen den schalenartigen Vertiefungen erlauben eine signifikante Verbesserung des mechanischen Verhakens zwischen dem Titansubstrat und einer darauf aufgebrachten Schicht bestehend z.B. aus Kunststoff. Die Hinterschnitte sowie die Submikro-Strukturen fungieren hierbei als Widerhaken.

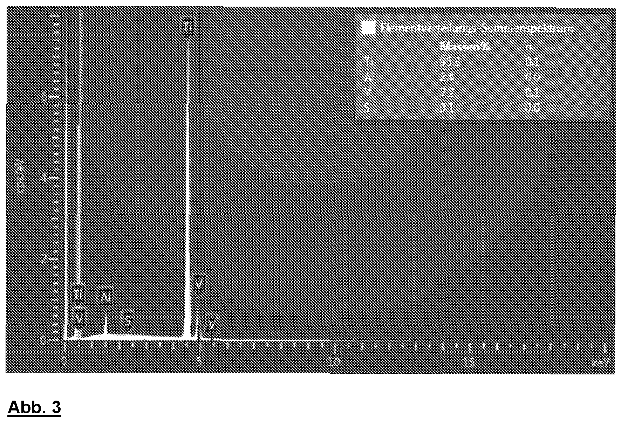

Abb. 3 zeigt das Spektrum der Elementen-Verteilung auf der Ti-3Al-2.5V Oberfläche. Es zeigt, dass nach der Prozessierung der oberflächennahe Aluminium-Anteil der Legierung um etwa 20% verringert wurde, während sich der Vanadium-Anteil wesentlich weniger verringert hat. Das Schwefelsignal ist vermutlich ein Artefakt bedingt durch das nicht ausreichend gründliche Spülen der Probe nach dem Ätzprozess.

### Beispiel 2: Ti-6Al-4V

### Herstellungsprozess:

Die Probe wurde für 5 min in Aceton entfettet und gereinigt und an Luft getrocknet. Anschließend wird die Probe für 20 h in konzentrierter H₂SO₄ bei Raumtemperatur ohne externe Temperierung unter Beleuchtung mit einem ENFIS Uno Tag Red LED-Array betrieben bei einer nominalen Bestrahlungsstärke von 400mW (300 mA und einer Wellenlänge von 620 nm) im Abstand von etwa 8 cm von der Probenoberfläche geätzt. Nach diesem Schritt wurde die Probe mehrfach in deionisiertem Wasser gereinigt und an Luft getrocknet. Anschließend wird die Probe für 3h in kurz vorher frisch hergestellter Ätzlösung bestehend aus HCl und H₂SO₄ im Volumen-Verhältnis 1:2 ohne externe Temperierung geätzt. Danach wurde die Probe mehrfach in deionisiertem Wasser gereinigt und an Luft getrocknet. Die Volumina der jeweiligen Ätzlösung wurden derart gewählt, dass eine vollständige Benetzung der Probe gegeben ist. Die Ätzlösungen wurden während der Ätzung nicht gerührt.

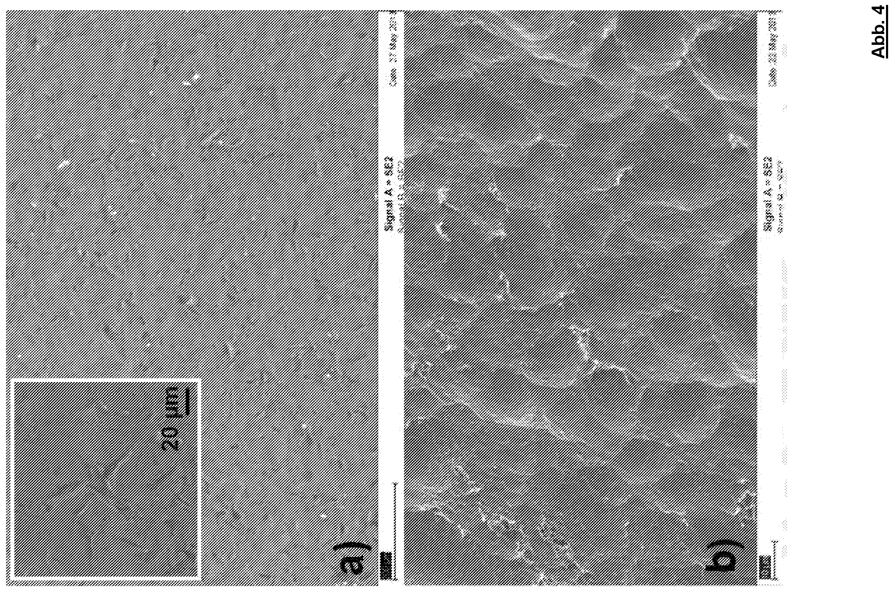

Die REM-Bilder sind in Abb. 4 gezeigt. Abb. 4a zeigt die Probe vor der erfindungsgemäßen Behandlung, es ist eine gestrahlte Ti-6Al-4V Oberfläche zu sehen. Man erkennt viele Scharten und tiefe Kratzer mit einer Länge von typischerweise etwa 20 µm - siehe Ausschnitt oben rechts Abb. 4a. Diese Oberflächendefekte können als Nukleationskeim für Risse fungieren, was schlimmsten Fall zum Versagen des Werkstückes führen kann. Abb 4b zeigt die Ti-6Al-4V Oberfläche nach erfindungsgemäßer Behandlung. Die Oberfläche zeigt eine schwach schalenartige Oberfläche mit einer glatten Nanostruktur. Die noch in Abb. 4a deutlich zu erkennbaren Oberflächendefekte wurden durch die Prozessierung vollständig entfernt.

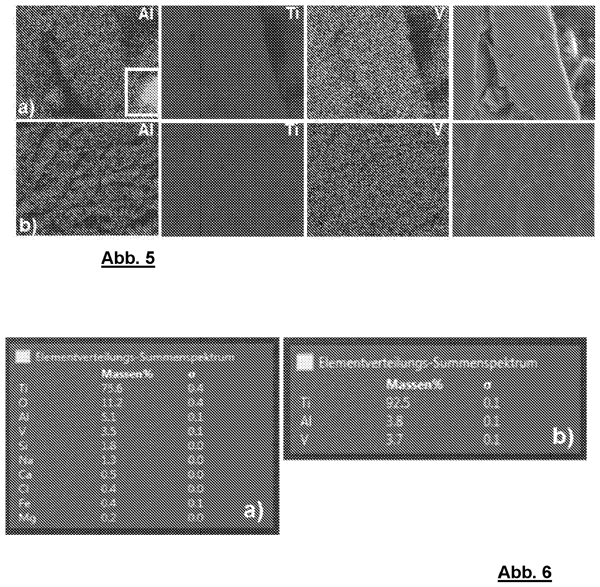

Abb 5. zeigt das EDX Element-Mapping von Ti, AI und V der zwei in Abb. 4 gezeigten Ti-6Al-4V Oberflächen. In Abb. 5a erkennt man im weiß gerahmten Bereich sehr AI-reiche Bereiche, höchstwahrscheinlich als Körner intermetallischer Phasen. Diese Körner können ebenfalls als Nukleationskeim für Risse fungieren, zusätzlich zu den in Abb. 4a gezeigten Scharten und Kratzern. Nach erfindungsgemäßer Prozessierung sind keine Al-reichen Bereiche mehr auf der Oberfläche zu finden.

Die integrale oberflächennahe Elementverteilung der gestrahlten Probe (Abb. 6a) zeigt vergleichsweise hohe Anteile an Alkalimetallen sowie Fe und Si. Diese können z.B. Rückstände des Strahlprozesses sein. Nach der erfindungsgemäßen Prozessierung sind alle Rückstände von Alkalimetallen sowie Fe und Si entfernt. Im Vergleich zur nicht erfindungsgemäß prozessierten Oberfläche ist die Konzentration an AI sehr stark reduziert, wobei der V Anteil gleichbleibt.

### Beispiel 3: Ti-6Al-4V (Ätzlösung ii mit Wasseranteil)

### Herstellungsprozess:

Die Probe wird für 5 min in Aceton gereinigt und entfettet und an Luft getrocknet. Anschließend wird die Probe für 20 h in konzentrierter H₂SO₄ bei Raumtemperatur ohne externe Temperierung unter Beleuchtung mit einem ENFIS Uno Tag Red LED-Array betrieben bei 300 mA (400 mW) im Abstand von etwa 8 cm von der Probenoberfläche geätzt. Nach diesem Schritt wurde die Probe mehrfach in deionisiertem Wasser gereinigt und an Luft getrocknet. Anschließend wird die Probe für 3h in kurz vorher frisch hergestellter Ätzlösung bestehend aus HCl, H₂SO₄, H₂O (deionisiert) im Volumen-Verhältnis 1:1:1 ohne externe Temperierung geätzt. Danach wurde die Probe mehrfach in deionisiertem Wasser gereinigt und an Luft getrocknet. Die Volumina der jeweiligen Ätzlösung wurden derart gewählt, dass eine vollständige Benetzung der Probe gegeben ist. Die Ätzlösungen wurden während der Ätzung nicht gerührt.

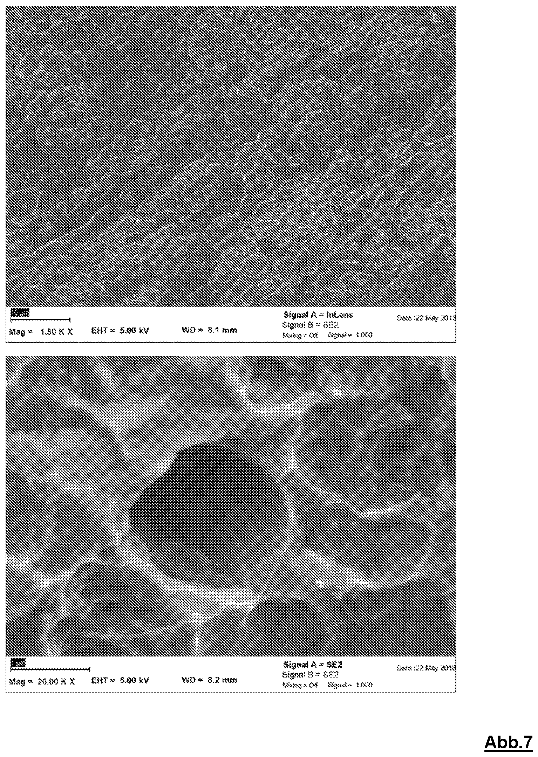

Abb 7 a) zeigt die Ti-6Al-4V Oberfläche nach dem zweistufigen Ätz-Prozess. Es ist eine feinausgeprägte schalenartige Oberfläche mit einer rauen Nanostruktur zuerkennen. Die Durchmesser der Schalen betragen etwa 2 µm. Teilweise sind diese Strukturen auch ineinander gewachsen. Abb. 7 b) zeigt eine solche schalenartige Struktur mit ihren nanostrukturierten Wänden. Im Gegensatz zu Abb 4 b) sind die schalenartigen Strukturen jedoch wesentlich kleiner im Durchmesser und deutlich flacher. Zusätzlichen weisen sie einen leichteren Hinterschnitt auf, wie in Abb. 7 b) zu sehen ist. Die in Abb 4 a) gesehenen Oberflächendefekte wurden durch die Prozessierung vollständig entfernt.

### Beispiel 4 Ti-6Al-4V (Ätzlösung i mit Wasseranteil)

### Herstellungsprozess:

Die Probe wird für 5 min in Aceton gereinigt und an Luft getrocknet. Anschließend wird die Probe für 20 h in einer Ätzlösung bestehend aus konzentrierter H₂SO₄ und deionisiertem Wasser (Volumenverhältnis 1:1) bei Raumtemperatur ohne externe Temperierung unter Beleuchtung mit einem ENFIS Uno Tag Red LED-Array betrieben bei 300 mA (400 mW) im Abstand von etwa 8 cm von der Probenoberfläche geätzt. Nach diesem Schritt wurde die Probe mehrfach in deionisiertem Wasser gereinigt und an Luft getrocknet. Anschließend wird die Probe für 3h in einem kurz vorher frisch hergestellter Ätzlösung bestehend aus konz. HCl und konz. H₂SO₄ im Volumen-Verhältnis 1:1 ohne externe Temperierung. Danach wurde die Probe mehrfach in deionisiertem Wasser gereinigt und an Luft getrocknet. Die Volumina der jeweiligen Ätzlösung wurden derart gewählt, dass eine vollständige Benetzung der Probe gegeben ist. Die Ätzlösungen wurden während der Ätzung nicht gerührt.

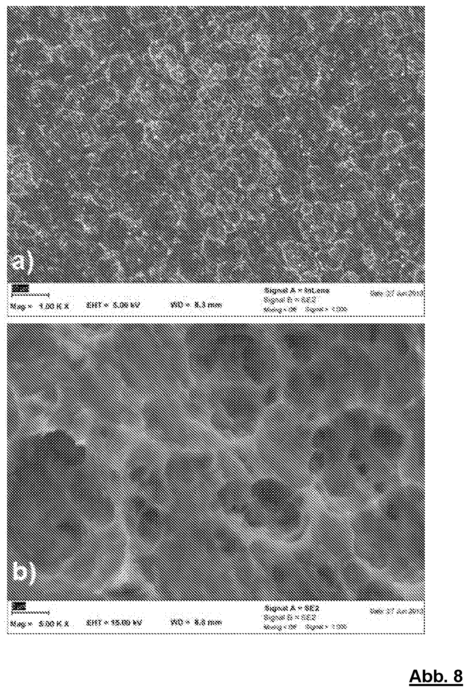

Abb 8 a) zeigt die Ti-6Al-4V Oberfläche nach dem zweistufigen Ätz-Prozess. Es ist eine feinausgeprägte schalenartige Oberfläche mit einer raueren Nanostruktur als in Abb. 7 a) zu sehen. Die Durchmesser der Schalen betragen etwa 4 µm. Teilweise sind diese Strukturen auch ineinander gewachsen. Abb. 8 b) zeigt eine solche schalenartige Struktur bestehend mehreren kleineren Schalen und nanostrukturierten Wänden. Im Gegensatz zu Abb. 7 b) sind die schalenartigen Strukturen jedoch wesentlich kleiner im Durchmesser und deutlich flacher. Zusätzlichen weisen sie einen leichten Hinterschnitt auf, wie in Abb. 8 b) zu sehen ist. Die in Abb. 4 a) gesehenen Oberflächendefekte wurden durch die Prozessierung vollständig entfernt.

### Beispiel 5: NiTi

### Herstellungsprozess:

Die Probe wurde für 5 min in Aceton entfettet und gereinigt und an Luft getrocknet. Anschließend wird die Probe für 20 h in konzentrierter H₂SO₄ bei Raumtemperatur ohne externe Temperierung unter Beleuchtung mit einem ENFIS Uno Tag Red LED-Array betrieben bei einer Bestrahlungsstärke von 200 mW (200 mA) im Abstand von etwa 8 cm von der Probenoberfläche geätzt. Da der NiTi-Draht von allen Seiten gleichzeitig strukturiert werden sollte, wurde der NiTi-Draht mit etwa 10 U/min um die eigene Achse gedreht bei stationärer, einseitiger Beleuchtung. Nach diesem Schritt wurde die Probe mehrfach in deionisiertem Wasser gereinigt und an Luft getrocknet. Anschließend wird die Probe für 2h in kurz zuvor frisch hergestellter Ätzlösung bestehend aus HCl und H₂SO₄ im Volumen-Verhältnis 1:2 ohne externe Temperierung geätzt. Danach wurde die Probe mehrfach in deionisiertem Wasser gereinigt und an Luft getrocknet. Die Volumina der jeweiligen Ätzlösung wurden derart gewählt, dass eine vollständige Benetzung der Probe gegeben ist. Die Ätzlösungen wurden während der Ätzung nicht gerührt.

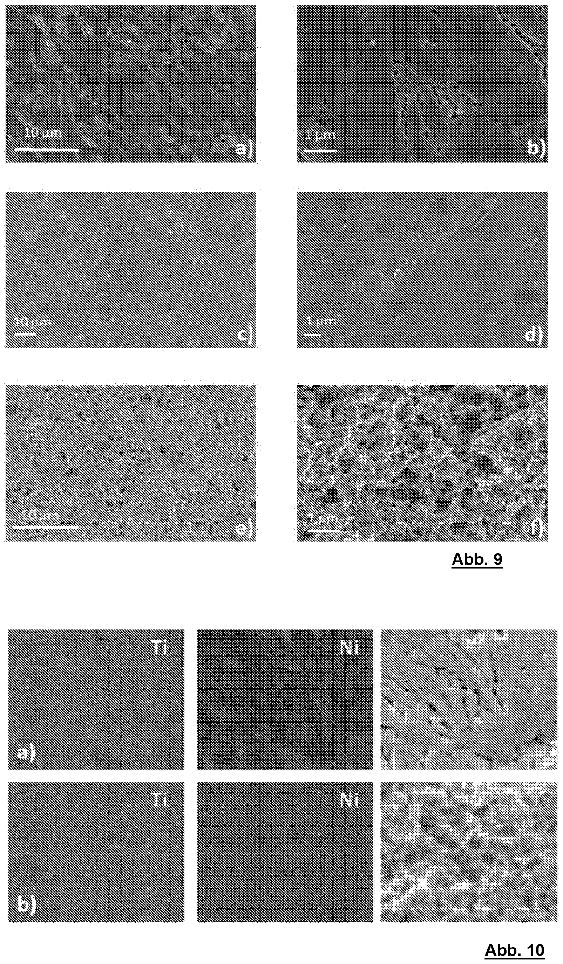

Die REM Bilder in Abb. 9 zeigen die Oberfläche von Drähten bestehend aus einer NiTi Legierung. Abb. 9 a und b zeigen die unbehandelte Oberfläche. Sie ist durchzogen von vielen tiefen, aber schmalen Furchen deren Form unregelmäßig und meist länglich gezogen ist. Der Ursprung dieser Furchen liegt daher vermutlich im Herstellungsprozess des Drahtziehens. Abb. 9 c und d zeigen die Oberflächenstruktur nach dem ersten Ätzschritt i. Die Oberfläche weist keinerlei Furchen mehr auf und sie ist wesentlich glatter im Vergleich zu der unbehandelten Oberfläche. An Stellen mit tiefen Furchen sind diese fast vollständig eingeebnet. Abb. 9 e und f zeigen die erfindungsgemäß komplett geänderte Oberfläche. Sie weist nun viele scharfe Kanten und Ecken auf, sowie schalenartige Vertiefungen, die aus mehreren mit Hinterschnitten versehenen "Löchern" bestehen. Einige dieser "Löcher" sind ineinander gewachsen. Die Oberfläche erscheint homogen in Bezug auf die sich eingestellte Rauigkeit.

Abb. 10 zeigt die Elementenverteilung von Ti, Ni auf der zusätzlich abgebildeten Probenoberfläche. Man erkennt in der unbehandelten Probe eine strukturelle Abhängigkeit zwischen der Nickelkonzentration an der Oberfläche und der Oberflächenstruktur (Abb. 10a). Dies gilt auch in abgeschwächter Form für Ti. Nach der erfindungsgemäßen Prozessierung ist dieser Zusammenhang zwischen Struktur und Ni/Ti Konzentration nicht mehr vorhanden. Die Oberfläche ist homogen in Bezug auf die Ni und Ti Verteilung.

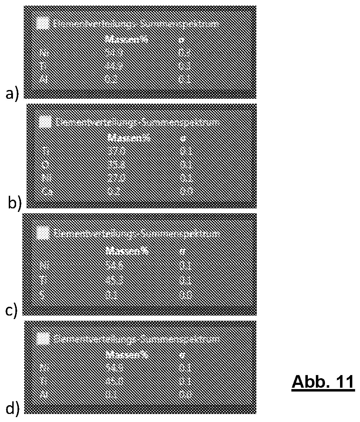

Das Summenspektrum der behandelten und unbehandelten Probe ist in Abb. 11 dargestellt. Im Volumen des NiTi Drahtes ist die Konzentration an Ni deutlich höher als die von Ti (Abb. 11a). Zusätzlich gibt es eine geringe Konzentration an AI. An der Oberfläche kehrt sich dieses Verhältnis um. Es ist mehr Ti als Ni vorhanden (Abb. 11b). Zusätzlich ist die Oberfläche stark oxidiert und es befindet sich Ca an der Oberfläche. Nach dem ersten Ätzschritt (Abb. 11c) kehrt sich das Verhältnis zwischen Ni und Ti an der Oberfläche wieder um und nähert sich den Bulk-Werten an. Nach vollständiger erfindungsgemäßer Prozessierung ist nahezu kein Unterschied zwischen Bulk und Oberfläche mehr feststellbar (Abb. 11d).

### Beispiel 6: Ti (grade 2)

### Herstellungsprozess:

Die Probe wurde mit Schleifpapier (4000 SiC-Körnung) so lange geschliffen, bis die Sägespuren entfernt waren und anschließend für 5 min in Aceton entfettet und gereinigt und an Luft getrocknet. Anschließend wird die Probe für 20 h in konzentrierter H₂SO₄ bei Raumtemperatur ohne externe Temperierung unter Beleuchtung mit einem ENFIS Uno Tag Red LED-Array betrieben bei einer nominalen Bestrahlungsstärke von 400mW (300 mA und einer Wellenlänge von 620 nm) im Abstand von etwa 8 cm von der Probenoberfläche geätzt. Nach diesem Schritt wurde die Probe mehrfach in deionisiertem Wasser gereinigt und an Luft getrocknet. Anschließend wird die Probe für 3h in kurz zuvor frisch hergestellter Ätzlösung bestehend aus HCl und H₂SO₄ im Volumen-Verhältnis 1:1 ohne externe Temperierung geätzt. Danach wurde die Probe mehrfach in deionisiertem Wasser gereinigt und an Luft getrocknet. Die Volumina der jeweiligen Ätzlösung wurden derart gewählt, dass eine vollständige Benetzung der Probe gegeben ist. Die Ätzlösungen wurden während der Ätzung nicht gerührt.

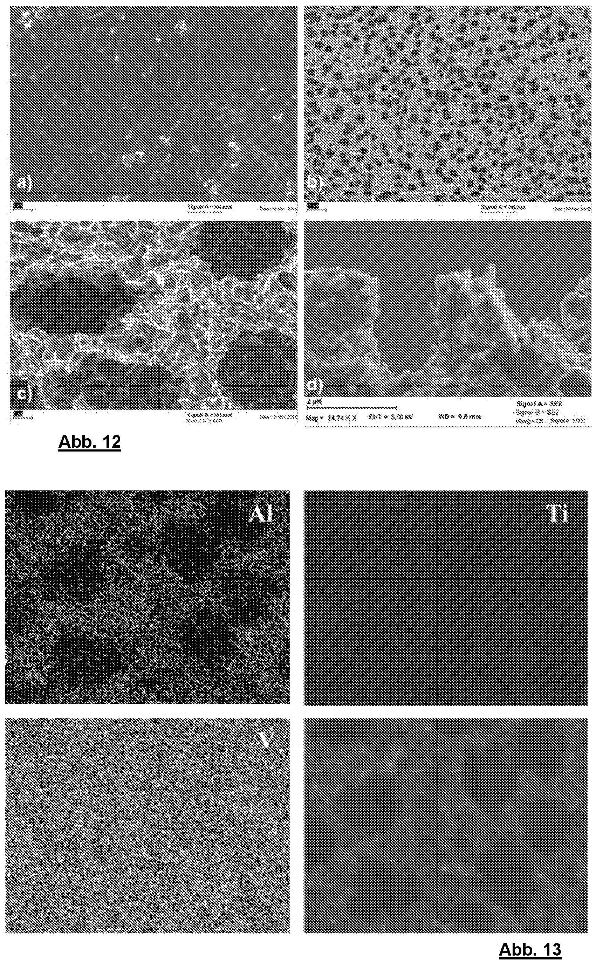

Abb. 12 zeigt die Oberfläche von Ti (grade 2) nach den einzelnen Ätzschritten. Abb. 12 a) zeigt eine glatte Oberfläche nach dem photochemischen Ätzschritt i. Die hellen Punkte sind Ablagerung von Salzen auf der Oberfläche aufgrund nicht ausreichend langer Reinigung. Die sich nach vollständiger erfindungsgemäßer Prozessierung ergebene Oberfläche ist in Abb. 12 b gezeigt. Die Oberfläche weist eine hohe Dichte an Poren auf. Teilweise beginnen diese schon ineinander zu wachsen. Ihre Grundform ist rundlich/oval mit unregelmäßig geformter Kante. Abb. 12 c zeigt, dass diese Poren an der Wand und an der Porenspitze scharfkantige Submikro-Strukturen aufweisen. Dieses ist auch gezeigt im Querschnitt in Abb. 12 d. Diese Poren weisen typischerweise einen Hinterschnitt auf. Dies ist zusammen mit den scharfkantigen Submikrostrukturen an der Porenwand von Vorteil für die mechanische Verzahnung mit einer auf die Oberfläche aufgebrachten Schicht z.B. ein Polymer.

Die EDX Element-Verteilung in Abb. 13 zeigt, dass es im Bereich der Poren zu einer lokalen Verarmung an AI kommt, während die Ti und V Konzentration davon unbeeinflusst sind. Wenn z.B. intermetallische Phasen wie Ti3AI an der Oberfläche vorhanden sind, werden diese selektiv gegenüber der Wirtsmatrix (Ti plus Verunreinigungen als Mischkristall) herausgeätzt. Infolge dessen entstehen die Poren an der Oberfläche.

### Beispiel 7 Ti (grade 2) Beleuchtung während des Ätzschrittes i im UV-Bereich

### Herstellungsprozess:

Die Probe wurde mit Schleifpapier (4000 SiC-Körnung) so lange geschliffen, bis die Sägespuren entfernt waren und anschließend für 5 min in Aceton gereinigt und an Luft getrocknet. Anschließend wird die Probe für 24 h in konzentrierter H₂SO₄ bei Raumtemperatur ohne externe Temperierung unter Beleuchtung mit einem ENFIS Uno Tag UV LED-Array betrieben bei 300 mA im Abstand von etwa 8 cm von der Probenoberfläche geätzt. Nach diesem Schritt wurde die Probe mehrfach in deionisiertem Wasser gereinigt und an Luft getrocknet. Anschließend wird die Probe für 3h in kurz vorher frisch hergestellter Ätzlösung bestehend aus HCl und H₂SO₄ im Volumen-Verhältnis 1:1 ohne externe Temperierung geätzt. Danach wurde die Probe mehrfach in deionisiertem Wasser gereinigt und an Luft getrocknet. Die Volumina der jeweiligen Ätzlösung wurden derart gewählt, dass eine vollständige Benetzung der Probe gegeben ist. Die Ätzlösungen wurden während der Ätzung nicht gerührt.

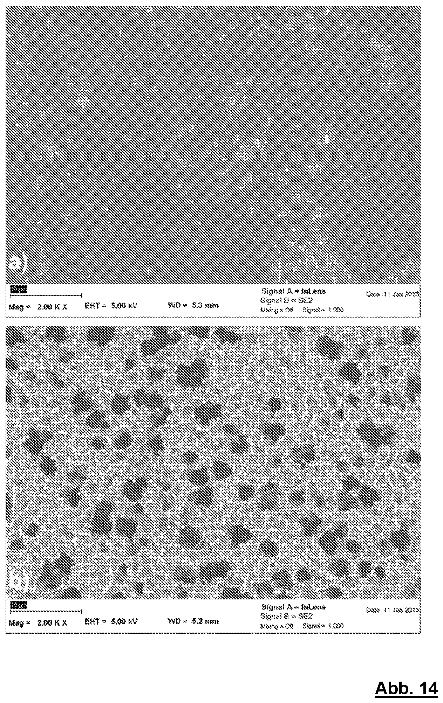

Abb. 14 zeigt die Oberfläche von Ti grade 2 nach den einzelnen Ätz-Schritten. Abb. 14 a zeigt eine glatte Oberfläche nach dem Ätzschritt i. Die hellen Punkte sind Ablagerung von Salzen auf der Oberfläche aufgrund nicht ausreichend langer Reinigung. Die sich nach vollständiger Prozessierung ergebene Oberfläche ist in Abb. 14 b gezeigt. Die Oberfläche weist eine hohe Dichte an Poren auf. Teilweise beginnen diese schon ineinander zu wachsen. Im Vergleich zu Abb.12b sind die Poren gezackter in ihrer Form.

## Patentansprüche

1. Oberflächenbehandeltes metallisches Werkstück aus Titan und/oder Titan-Legierungen mit Titan als Hauptbestandteil und/oder Nickel-Titan-Legierungen sowie Nitinol,
**dadurch gekennzeichnet, dass**
- das Metall an der behandelten Oberfläche frei von Einschlüssen, Ausscheidungen anderer Metalle, Anlagerungen von Alkali-, Erdalkalimetallen und/oder Aluminium, intermetallischen Phasen, und/oder mechanisch stark defektreichen Bereichen ist,
und
die Oberfläche über eine erste Rauigkeit und eine zweite Rauigkeit verfügt,
wobei
die erste Rauigkeit durch Vertiefungen in Form von Poren gegeben ist,
wobei die Poren
- einen Durchmesser im Bereich zwischen 0,5 und 50 µm haben
- in Richtung der Oberfläche offen
und
- in Richtung des Werkstücks geschlossen sind,
und wenigstens ein Teil der Poren einen Hinterschnitt aufweisen und die zweite Rauigkeit durch statistisch verteilte Erhöhungen und Vertiefungen im Bereich von 100nm und weniger gegeben ist.

2. Herstellungsverfahren des oberflächenbehandelten Werkstücks nach Anspruch 1,
umfassend die kombinierten und nachfolgend hintereinander auszuführenden Schritte:
i. photochemisches Ätzen in Gegenwart eines sauren Elektrolyten bei gleichzeitigem Beleuchten, wobei der saure Elektrolyt eine stark oxidierende Säure ist
und
ii. chemisches Ätzen in saurer Lösung, wobei die saure Lösung eine Kombination aus einer stark oxidierenden Säure und einer oxidauflösenden Säure ist.

3. Herstellungsverfahren nach Anspruch 2,
**dadurch gekennzeichnet, dass**
das Beleuchten bei einer Beleuchtungsstärke von 200 bis 450 mW und einer Wellenlänge zwischen 190 und 780 nm erfolgt.

4. Herstellungsverfahren nach Anspruch 2 oder 3,
**dadurch gekennzeichnet, dass**
zwischen dem i.-Ätzen und dem ii.-Ätzen oder nach dem i.-Ätzen und dem ii.-Ätzen ein Spülen und Trocknen erfolgt.

5. Herstellungsverfahren nach Anspruch 2, 3, oder 4,
**dadurch gekennzeichnet, dass**
die stark oxidierende Säure ausgewählt aus der Gruppe der Sauerstoffsäuren ist.

6. Herstellungsverfahren nach Anspruch 2, 3, 4 oder 5,
**dadurch gekennzeichnet, dass**
die stark oxidierende Säure Schwefelsäure (H₂SO₄), Salpetersäure (HNO₃), Peroxomonoschwefelsäure (H₂SO₅), Chlorsäure (HClO₃), Perchlorsäure (HClO₄), Chromsäure (H₂CrO₄), Arsensäure (H₃AsO₄) und/oder Wasserstoffperoxid (H₂O₂) ist.

7. Herstellungsverfahren nach einem der Ansprüche 2 bis 6,
**dadurch gekennzeichnet, dass**
die oxidauflösende Säure Salzsäure (HCl), Flusssäure (HF), Bromsäure (HBr) und/oder Iodsäure (HI) ist.

8. Herstellungsverfahren nach Anspruch 5, 6 oder 7,
**dadurch gekennzeichnet, dass**
die stark oxidierende Säure oder die oxidauflösende Säure in Verdünnung mit Wasser (H₂O) vorliegt.

## Claims

1. A surface-treated metallic workpiece of titanium and/or titanium alloys with titanium as the main component and/or nickel-titanium alloys as well as Nitinol,
**characterised in that**
- the metal on the treated surface is free of inclusions, precipitations of other metals, deposits of alkali metals, alkaline earth metals and/or aluminium, intermetallic phases and/or areas which are mechanically highly defective,
and
the surface has a first roughness and a second roughness,
wherein
the first roughness results from depressions in the form of pores,
wherein the pores
- have a diameter in the range of between 0.5 and 50 µm
- are open towards the surface
and
- are closed towards the workpiece,
and at least some of the pores have an undercut section and the second roughness results from statistically distributed elevations and depressions in the range of 100 nm and less.

2. A production method for the surface-treated workpiece according to claim 1,
comprising the following combined steps to be performed successively:
i. photochemical etching in the presence of an acidic electrolyte with simultaneous illumination, the acidic electrolyte being a strongly oxidising acid,
and
ii. chemical etching in an acidic solution, the acidic solution being a combination of a strongly oxidising acid and an oxide-dissolving acid.

3. The production method according to claim 2,
**characterised in that**
the illumination takes place at an illumination intensity of 200 to 450 mW and a wavelength of between 190 and 780 nm.

4. The production method according to claim 2 or 3,
**characterised in that**
between the step i. etching and the step ii. etching, or after the step i. etching and the step ii. etching, rinsing and drying take place.

5. The production method according to claim 2, 3 or 4,
**characterised in that**
the strongly oxidising acid is selected from the group of the oxyacids.

6. The production method according to claim 2, 3, 4 or 5,
**characterised in that**
the strongly oxidising acid is sulfuric acid (H₂SO₄), nitric acid (HNO₃), peroxymonosulfuric acid (H₂SO₅), chloric acid (HClO₃), perchloric acid (HClO₄), chromic acid (H₂CrO₄), arsenic acid (H₃AsO₄) and/or hydrogen peroxide (H₂O₂).

7. The production method according to one of claims 2 to 6,
**characterised in that**
the oxide-dissolving acid is hydrochloric acid (HCI), hydrofluoric acid (HF), bromic acid (HBr) and/or iodic acid (HI).

8. The method according to claim 5, 6 or 7,
**characterised in that**
the strongly oxidising acid or the oxide-dissolving acid is present in dilution with water (H₂O).

## Revendications

1. Pièce à usiner métallique traitée en surface constituée de titane et/ou d'alliages de titane avec du titane comme composant principal et/ou d'alliages nickel-titane ainsi que de nitinol,
**caractérisé en ce que**
- le métal, au niveau de la surface traitée, est exempt d'inclusions, de précipitations d'autres métaux, de dépôts de métaux alcalins, métaux alcalino-terreux et/ou aluminium, de phases intermétalliques et/ou de zones très riches en défauts mécaniques,
et
la surface présente une première rugosité et une deuxième rugosité,
la première rugosité étant obtenue par des creux sous forme de pores,
les pores
- ayant un diamètre dans la fourchette comprise entre 0,5 et 50 µm,
- étant ouverts en direction de la surface,
et
- étant fermés en direction de la pièce à usiner,
et au moins une partie des pores présente une contre-dépouille,
et la deuxième rugosité est obtenue par des bosses et des creux statistiquement distribués et dans la fourchette de 100 nm et moins.

2. Procédé de fabrication de la pièce à usiner traitée en surface selon la revendication 1,
comprenant les étapes combinées et à effectuer successivement consistant à :
i. réaliser une gravure photochimique en présence d'un électrolyte acide avec éclairage concomitant, l'électrolyte acide étant un acide fortement oxydant,
et
ii. réaliser une gravure chimique en solution acide, la solution acide étant une combinaison d'un acide fortement oxydant et d'une d'un acide solubilisant les oxydes.

3. Procédé de fabrication selon la revendication 2,
**caractérisé en ce que**
l'éclairage est réalisé avec une intensité lumineuse de 200 à 450 mW et une longueur d'onde comprise entre 190 et 780 nm.

4. Procédé de fabrication selon la revendication 2 ou 3,
**caractérisé en ce qu'**
un rinçage et séchage est réalisé entre le la gravure i. et la gravure ii. ou après la gravure i. et la gravure ii.

5. Procédé de fabrication selon la revendication 2, 3 ou 4,
**caractérisé en ce que**
l'acide fortement oxydant est choisi dans le groupe des oxacides.

6. Procédé de fabrication selon la revendication 2, 3, 4 ou 5,
**caractérisé en ce que**
l'acide fortement oxydant est l'acide sulfurique (H₂S0₄), l'acide nitrique (HN0₃), l'acide peroxymonosulfurique (H₂S0₅), l'acide chlorique (HClO₃), l'acide perchlorique (HClO₄), l'acide chromique (H₂CrO₄), l'acide arsénique (H₃As0₄) et/ou le peroxyde d'hydrogène (H₂O₂).

7. Procédé de fabrication selon l'une des revendications 2 à 6,
**caractérisé en ce que**
l'acide solubilisant les oxydes est l'acide chlorhydrique (HCl), l'acide fluorhydrique (HF), l'acide bromique (HBr) et/ou l'acide iodrique (HI).

8. Procédé de fabrication selon la revendication 5, 6 ou 7, **caractérisé en ce que** l'acide fortement oxydant ou l'acide solubilisant les oxydes est présent en dilution avec de l'eau (H₂O).
